# EUROPÄISCHE PATENTSCHRIFT

(11) **EP 3 500 198 B1**
(45) Veröffentlichungstag und Bekanntmachung des Hinweises auf die Patenterteilung: **18.03.2020**
(21) Anmeldenummer: 17772600.7
(22) Anmeldetag: 16.08.2017
(51) Int. Cl.: A61B 18/02, A61B 5/0215, A61B 5/20, A61B 18/00

(54) **MEDIZINISCHES GERAET ZUR DENERVIERUNG RENALER PERIVASKULAERER NERVEN**
MEDICAL APPLIANCE FOR DENERVATION OF RENAL PERIVASCULAR NERVES
APPAREIL MÉDICAL POUR DÉNERVER DES NERFS PÉRIVASCULAIRES RÉNAUX

(30) Priorität: 18.08.2016 DE 102016115387
(43) Veröffentlichungstag der Anmeldung: 26.06.2019
(73) Patentinhaber: Cardiolectra GmbH, 10119 Berlin (DE)
(72) Erfinder: KEWELOH, Boris, 10119 Berlin (DE)
(74) Vertreter: Stütz, Jan
(86) Internationale Anmeldenummer: PCT/DE2017/100691
(87) Internationale Veröffentlichungsnummer: WO 2018/033186

(56) Entgegenhaltungen:
- DE-U1-202016 104 549
- US-A1- 2009 299 355
- US-A1- 2011 264 086
- US-A1- 2013 131 743
- US-A1- 2014 249 520
- US-A1- 2015 066 007

## Beschreibung

Die Erfindung betrifft ein medizinisches Gerät zur Denervierung renaler perivaskulärer Nerven.

Nach dem Stand der Technik werden derartige Geräte eingesetzt, um Patienten mit Hypertonie bzw. Bluthochdruck zu behandeln.

In den meisten Fällen lässt sich der Bluthochdruck durch eine angepasste Ernährung wie etwa die Reduzierung des Salz- und Alkoholkonsums sowie eine Steigerung der sportlichen Betätigung beseitigen oder reduzieren. Auch die Reduzierung eines Übergewichts und die Vermeidung von Stress können den Blutdruck senken. Bei Patienten mit höherem Blutdruck kommen pharmazeutische Präparate zum Einsatz, welche in der richtigen Dosierung bzw. bei richtiger Einstellung des Patienten dauerhaft den Blutdruck in akzeptablen Grenzen halten.

Es gibt aber auch Patienten, bei denen eine nachhaltige Blutdrucksenkung mit langfristig anwendbaren Präparaten nicht möglich ist.

Diese Patienten leiden meist an einer besonders stark ausgeprägten Hyperaktivität des sympathischen Nervensystems.

Es hat sich gezeigt, dass trotz einer bestehenden Multikausalität der Hypertonie, die Aktivität der renalen perivaskulären Nerven eine große Rolle spielt.

Insbesondere Patienten bei denen es nicht möglich ist, mit Hilfe von pharmazeutischen Präparaten den Blutdruck dauerhaft auf ein akzeptables Niveau einzustellen, werden aus diesem Grund bereits mit medizinischen Geräten zur Denervierung der renalen perivaskulären Nerven behandelt.

Bislang hat die Denervierung der renalen perivaskulären Nerven nicht immer den gewünschten Erfolg.

Bei vielen Patienten fällt die Blutdrucksenkung nach einer Zeit von etwa 6 Monaten nach der Behandlung so gering aus, dass das Risiko eines invasiven Eingriffes in einem schlechten Verhältnis zu möglichen Heilungschancen steht.

Die Ursache für die eingeschränkte Wirksamkeit von Nierenablationsverfahren aus dem Stand der Technik war bislang nicht bekannt.

Folgende Dokumente zeigen Ballonkatheter aus dem Stand der Technik: US 2014/0249520 A1, US 2015/0066007 A1, US 2013/0131743 A1, US 2011/0264086 A1.

Die Aufgabe der Erfindung besteht darin, ein Gerät zu Verfügung zu stellen, welche die aus dem Stand der Technik bekannten Nachteile zumindest reduziert und eine nachhaltiger Senkung des Blutdrucks durch die renale Denervierung ermöglicht.

Diese Aufgabe wird mit einem medizinischen Gerät nach Anspruch 1 gelöst. Die Unteransprüche stellen bevorzugte Ausführungsformen der Erfindung dar.

Das erfindungsgemäße medizinische Gerät zur Denervierung renaler perivaskulärer Nerven verfügt dabei über einem Katheter mit einem flexiblen Schaft zum Einführen in die renale Arterie eines Patienten.

Der Schaft hat dabei ein inneres Lumen, welches zum Zuführen eines Kältemittels geeignet ist. Am distalen Ende des Schaftes weist das medizinische Gerät einen Kryoballon auf. Dieser Kryoballon steht mit dem Lumen des Schaftes in Verbindung und wird über den Schaft mit einem Kältemittel versorgt. Durch die Verdampfung des Kältemittels in dem Ballon wird auf der Oberfläche des Ballons eine Temperatur vorzugsweise zwischen - 30 und -80 °C erzeugt, mit deren Hilfe die renalen perivaskulären Nerven abladiert werden.

Auf der Oberfläche des Ballons ist wenigstens eine bipolare Elektrode angeordnet und mit einer Stromquelle zur Abgabe eines elektrischen Impulses oder einer Abfolge von Impulsen oder einer Frequenz zur Stimulation der perivaskulären Nerven verbunden.

Diese Anregung der perivaskulären Nerven wird für einen Feedbackloop verwendet, um den Behandlungserfolg zu überprüfen.

Dabei ist das erfindungsgemäße medizinische Gerät dadurch gekennzeichnet, dass der Kryoballon dazu ausgebildet ist, im Anwendungsfall die Wand der renalen Arterie (in Blutströmungsrichtung) auf einer Länge von mehr als 2,5 cm, vorzugsweise mindestens 3 cm und besonders bevorzugt wenigstens 3,5 cm zu berühren und auf einer Länge von mindestens 2,5 cm, vorzugsweise mindestens 3 cm und besonders bevorzugt wenigstens 3,5 cm (in Blutströmungsrichtung) durchgehend und gleichzeitig zu abladieren.

Im expandierten Zustand hat der Kryoballon einen Durchmesser von zwischen 3 und 7 mm vorzugsweise zwischen 4 und 6 mm.

Aus dem medizinischen Prinzip heraus, das geringstmögliche Gewebevolumen zu abladieren, um einen medizinischen Erfolg (hier die Ablation und Unterbrechung der Reizleitung der renalen perivaskulären Nerven) zu erzielen, ist das Vorsehen von einer derart großen Ablationsoberfläche bei Geräten aus dem Stand der Technik nicht erwünscht gewesen. Vor allem, da das Risiko einer Gefäßverengung, einer Blutgerinnselauflagerung und einer Gefäßperforation kritisch betrachtet werden muss.

Darüber hinaus wurde eine Unterbrechung der Reizleitung auch schon bei einer Ablation von bestimmten Bereichen in der renalen Arterie erzielt, sodass großflächige Ablationen gezielt vermieden wurden.

Umso überraschender war das Ergebnis, dass mit mehreren ausführlichen Kälteablationen der renalen perivaskulären Nerven auf möglichst der gesamten Länge der renalen Arterie und bevorzugt der gesamten Zirkumferenz eine nachhaltige Blutdrucksenkung bei den behandelten Patienten erzielt werden kann.

Das erfindungsgemäße medizinische Gerät ist dementsprechend von den geometrischen Abmessungen dazu ausgebildet, die Nierenarterie im Großteil ihrer Länge und bevorzugt in ihrer gesamten Zirkumferenz zu abladieren.

Außerdem verwendet das erfindungsgemäße medizinische Gerät Kälte (Kryoenergie) und nicht Radiofrequenzenergie (Hitze). Kälteablationen reißen die Gefäßinnenwand seltener ein, es kommt seltener zur Auflagerung von Blutgerinnseln und die Gefahr von Perforationen des Gefäßes ist geringer.

Im Anwendungsfall ist der Katheter, auf dem der Kryoballon angeordnet ist, mit einer Kältemittelquelle und einer Steuerung für die Kältemittelquelle verbunden.

Gemäß einer bevorzugten Ausführungsform wird dabei die Kältemittelquelle bzw. die Zufuhr des Kältemittels in den Kryoballon in Abhängigkeit von einem Blutdruckwert des Patienten während der Behandlung gesteuert.
Nach einer weiteren Ausführungsform verfügt der Katheter über einen Drucksensor. Hierdurch kann während der Denervierung eine direkte (invasive) Blutdruckmessung durchgeführt werden. Der Blutdruckwert wird dabei erfasst und der Steuerung zugeführt, welche in Abhängigkeit von diesem Wert die Kältemittelquelle bzw. die Zufuhr des Kältemittels zum Kryoballon steuert.

Es ist aber auch möglich, dass die Steuerung mit einer Blutdruckmanschette verbunden ist und der aktuelle Blutdruck dementsprechend indirekt gemessen wird. Aber auch hier findet eine Steuerung der Kältemittelquelle bzw. die Zufuhr des Kältemittels zum Kryoballon in Abhängigkeit von dem gemessenen Blutdruck statt.
Eine invasive Blutdruckmessung bietet jedoch noch weitere Vorteile: Vorzugsweise wird mit dem Sensor die Druckanstiegsgeschwindigkeit, also delta mmHg/ s gemessen. Besonders bevorzugt erfolgt dies innerhalb des Anstieges eines Schlages, als Maß der Herzkraft und des Blutdrucks. Hierdurch ist es möglich, viel früher und sicherer zu diagnostizieren, ob die renalen perivaskulären Nerven schon ausreichend abladiert sind oder noch stimuliert werden können.
Nach einer weiteren Ausgestaltung der Erfindung ist auch die Stromquelle zur Abgabe eines elektrischen Impulses oder einer elektrischen Frequenz über wenigstens eine Elektrode mit der Steuerung verbunden. Die Steuerung leitet dann zunächst die Abgabe eines oder einer Reihe von Impulsen oder eine Frequenz über wenigsten eine Elektrode ein. Dieser Vorgang kann auch als Anregungsphase bezeichnet werden.

Daraufhin prüft die Steuerung mit Hilfe des gemessenen Blutdrucks oder eines korrelierenden Wertes, ob eine Blutdruckerhöhung innerhalb eines vorgegebenen Zeitintervalls und oberhalb eines vorgegebenen Grenzwertes erfolgt ist.

Ist dies der Fall, dann wird eine Ablationsphase durch Ansteuerung der Kältemittelquelle eingeleitet oder eine Ablationsphase wird zumindest für eine vorherbestimmte Zeit fortgesetzt oder aber wiederholt.

Vorzugsweise wird in diesem Zusammenhang der Blutdruck oder ein korrelierender Wert, welcher kurz vor oder während der Anregung der perivaskulären Nerven mit Hilfe elektrischer Impulse gemessen wurde, mit dem Wert des Blutdrucks nach einer Zeit von zwischen 15 Sekunden und 5 Minuten, weiter bevorzugt nach einer Zeit von zwischen 2 und 4 Minuten nach Beginn der Anregungsphase verglichen.

Die Anregung der perivaskulären Nerven erfolgt z. B. mit einer Frequenz von 20HZ, einer Spannung von 15 Volt (Amplitude) und einer Länge von wenigstens 10ms.

Der Grenzwert der minimalen Druckerhöhung, bei der eine Ablationsphase eingeleitet oder aufrechterhalten wird, beträgt vorzugsweise 5 mmHG.

Die Ablationphase beträgt vorzugsweise zwischen 2 und 3 Minuten, wobei der Ballon an der Oberflächen bevorzugt eine Temperatur von unter -50 °C aufweist.

Beispielsweise wird die Ablationsphase beendet, wenn trotz einer Anregung, welche auch während der Ablationsphase stattfindet, der Blutdruck nicht über einen vor Beginn der Anregungsphase gemessenen Wert hinausgeht.
Nach Beendigung einer Ablationsphase ist die Steuerung vorzugsweise dazu ausgebildet, nach einer Zeit von zwischen 5 und 20 Minuten eine weitere Anregungs- und ggf. Ablationsphase zu starten.
Besonders bevorzugt startet die Steuerung zwischen 5 und 10 Minuten nach Beendigung einer Ablationsphase eine weitere Anregungsphase. Wird bei Anregung aber keine Blutdruckerhöhung um einen vorgegebenen Grenzwert erfasst, so startet die Steuerung nach erneut 5 bis 10 Minuten eine weitere Anregungsphase. Wird hierbei oder gemäß einer Abwandlung der Ausführungsform bei einer weiteren Anregungsphase weiterhin keine Blutdruckerhöhung festgestellt, so wird die Behandlung beendet. Ansonsten startet der Zyklus von vorne.
Gemäß einer weiteren Ausführungsform ist vorgesehen, dass die Elektroden Impedanz-Messungen vornehmen. Nach einer bevorzugten Ausführungsform wird die Impedanz-Messung von der Steuerung dazu verwendet, einen Wert für die Läsionstiefe zu ermitteln. Vorzugsweise ist zudem vorgesehen, dass in der Steuerung ein Grenzwert für die Läsionstiefe abgelegt ist. Entsprechend wird der Behandlungszyklus abgebrochen, wenn die mit Hilfe der Elektroden durchgeführte Impedanz-Messung einen Wert ergibt, welcher das Überschreiten einer zulässigen hiermit zusammenhängenden Läsionstiefe kennzeichnet.

Erfindungsgemäß ist der Kälteballon so ausgebildet, dass er einen an der Außenseite in Längsrichtung verlaufenden bevorzugt kälteisolierten Kanal aufweist, welcher sich radial zwischen 10 und 40 % seiner Zirkumferenz und weiter bevorzugt zwischen 25 und 35% seiner Zirkumferenz erstreckt. Der Kanal hat bevorzugt die Form eines Zylindersektors.
Durch diesen Kanal kann während der 2-3 Minuten andauernden Ablation Blut zur Niere strömen. Dies verhindert etwaige Schäden des Nierengewebes durch eine wiederholte Blutleere.

Beispielsweise weist der Kryoballon an der Stelle des zu bildenden Kanals einen Streifen nicht oder wenig dehnbaren Materials auf. Dieser Streifen, welcher auf den Katheter oder auch auf den Kryoballon geklebt sein kann oder teilweise das ursprüngliche dehnbare Material des Kryoballons ersetzt, verhindert eine Ausdehnung des Kryoballons an dieser Stelle, wodurch ein Kanal im Querschnitt der Arterie freigehalten wird, damit es zu einer Strömung von Blut zur Niere auch während der Behandlung kommt.
Wenn der Zylindersektor einen Radius von mehr als 180° aufspannt, kann gewährleistet werden, dass die gesamte Zirkumferenz der Gefäßwand mit dem Kryoballon bei einer zweimaligen Anwendung in Kontakt kommt, wenn der Kryoballon während der Behandlung wenigstens einmal um die Längsachse um einen Minimalwinkel gedreht wird, welcher größer, als der Winkel ist, welchen der Zylindersektor des Kanals 8 aufspannt.

Beispielsweise ist vorgesehen, dass der Kryoballon zwar zylinderförmig ausgebildet ist, jedoch einen Bypass-Kanal mit anderen Worten einen Tunnel durch den Ballon aufweist, welcher einen Blutfluss zur Niere auch während der Behandlung ermöglicht.

Weitere Details sind aus der Figurenbeschreibung zu entnehmen, welche bevorzugte Ausführungsformen der Erfindung darstellen.
Dabei zeigen:
- Fig. 1: das erfindungsgemäße medizinische Gerät mit Kältemittelquelle und Steuereinheit,
- Fig. 2: den Katheter in einer ersten Ausführungsform, die nicht Teil der Erfindung ist, in einer Seitenansicht,
- Fig. 3: den Katheter aus Figur 2 in einer Querschnittsdarstellung der Ebene A-A,
- Fig. 4: eine Querschnittsdarstellung einer Ausführungsform des Katheters,
- Fig. 5: eine Querschnittsdarstellung einer weiteren Ausführungsform des Katheters.

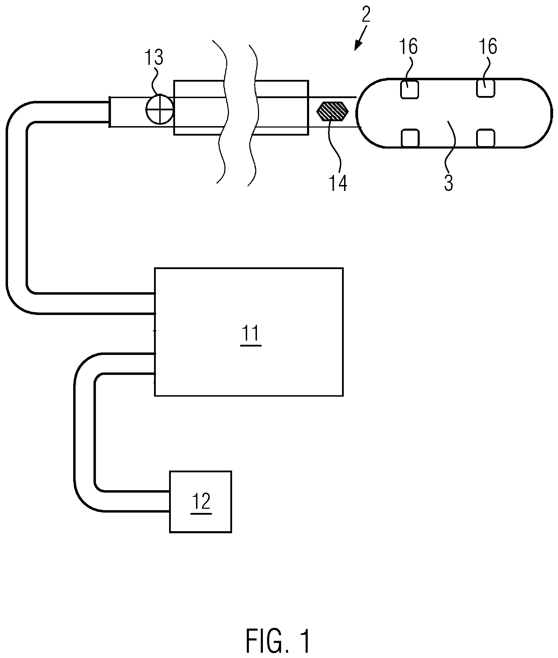

Figur 1 zeigt das erfindungsgemäße medizinische Gerät 1. Das Gerät umfasst dabei einen Katheter 2 mit einer flexiblen Mantelfläche, welcher zum Einführen in die Arterie eines Menschen und speziell in die renale Arterie ausgebildet ist.
Am distalen Ende ist der Katheter 2 expandierbar ausgebildet und bildet im Anwendungsfall einen Kryoballon 3 zum Abladieren von Körpergewebe.
Am Schaft des Katheters ist eine Vorrichtung zur Messung des Blutdrucks 14 angeordnet. In der Nähe des proximalen Endes des Katheters 2 ist eine Markierung 13 angebracht, mit deren Hilfe ein Drehen des Katheters um seine Längsachse während der Behandlung festgestellt werden kann.
Am proximalen Ende des Katheters sind eine elektrische Verbindung und eine Verbindung für ein Kältemittel vorgesehen, welche den Katheter 2 mit einer Steuerung 11 verbindet. Die Steuerung selbst ist zudem mit einer Quelle für ein Kältemittel 12 verbunden und steuert im Anwendungsfall die Zuführung von Kältemittel in den am distalen Ende des Katheters 2 angeordneten Kryoballon 3.

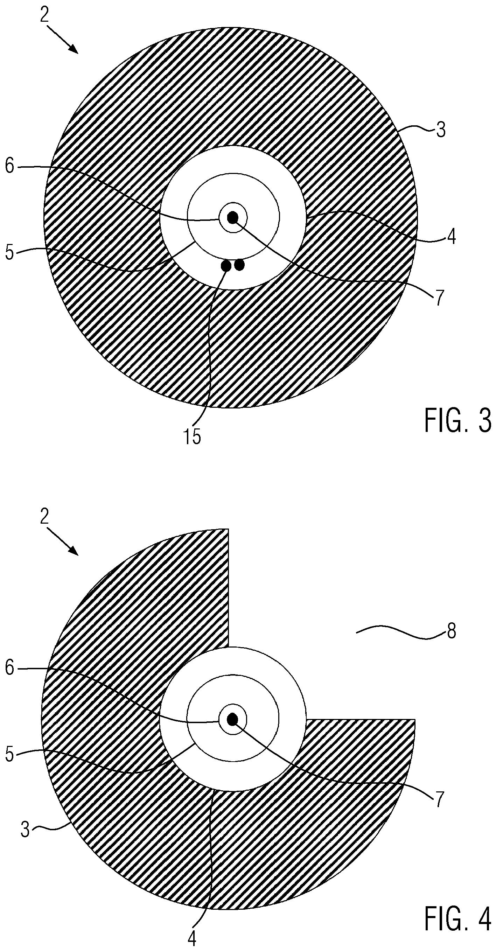

Über elektrische Leitungen 15 (siehe Figur 3) ist die Steuerung 11 zudem mit dem Drucksensor 14 zur Bestimmung des Blutdruckes verbunden. Weitere elektrische Leitungen verbinden vier auf dem Kryoballon 3 angeordnete Elektroden 16 mit der Steuerung 11.

Im Anwendungsfall stimuliert wenigstens eine der Elektroden 16 die renalen perivaskulären Nerven eines Patienten. Die Steuerung 11 stellt fest, ob auf die Stimulierung durch die Elektrode(n) 16 eine Blutdruckerhöhung oder die Erhöhung eines mit dem Blutdruck korrelierenden Wertes innerhalb eines vorherbestimmten Zeitintervalls und oberhalb eines vorgegebenen Grenzwertes stattgefunden hat.

Ist dies der Fall, so leitet die Steuerung 11 Kältemittel aus der Kältemittelquelle 12 über den Katheter 2 in den Kryoballon 3 und bewirkt eine Ablation der renalen perivaslukären Nerven. Eine Markierung 13 ist auf dem Katheter vorgesehen, welche gestattet, die Drehung des Katheters um seine Längsachse festzustellen und zu prüfen, ob eine gewünschte Mindestdrehung bei der Behandlung durchgeführt wurde.

Dies ist vor allem bei Ausführungsformen sinnvoll, bei denen der Kryoballon 3 so ausgebildet ist, dass er im Querschnitt und im Behandlungsfalle nicht die gesamte Zirkumferenz der arteriellen Gefäßwand berührt.

In diesem Falle ist es für die Behandlung notwendig, die Ablation der renalen perivaskulären Nerven in wenigstens zwei und bevorzugt genau zwei Behandlungsschritten durchzuführen. Der Katheter muss während der Behandlung dann wenigstens einmal um einen vorgegebenen Mindestwinkel um die Längsachse des Katheters 2 verdreht werden.

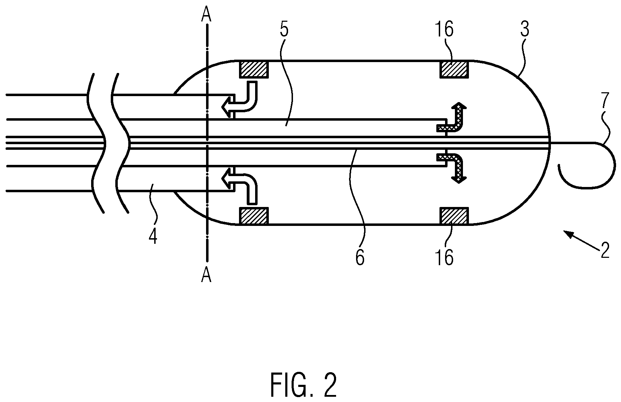

Figur 2 zeigt einen Längsschnitt durch den Katheter 2 mit symmetrischem Querschnitt. Der Katheter verfügt dabei über 3 Lumen. Das innerste Lumen 6 ist zur Aufnahme eines J-wires ausgebildet und entsprechend dimensioniert. Im Behandlungsfalle hilft der J-wire, den Katheter in die renale Arterie eines Patienten einzuführen. Sobald der Katheter 2 an seinem Bestimmungsort in der renalen Arterie des Patienten angelangt ist, wird über das äußerste Lumen 4 Druckluft in den Kryoballon 3 geleitet, wodurch dieser sich entfaltet und an die Gefäßwand der Arterie anlegt. Mittels der Elektroden 16 wird dann eine Stimulation der renalen perivaskulären Nerven bewirkt, was normalerweise zu einer Blutdruckerhöhung führt.
Wird eine Blutdruckerhöhung festgestellt, tritt flüssiges Kältemittel (bevorzugt N₂O) über das distale Ende des Lumens 5 in den Kryoballon 3 ein und verdampft. Durch die Verdampfung wird Kälte erzeugt, welche über die Wandung des Kryoballons 3 an das Gewebe abgegeben wird und durch Ablation zu einer Denervierung führt. Die expandierten Gase werden dann über das äußerste Lumen 4 zum proximalen Ende des Katheters 2 geleitet und an die Narkosegasabsaugung des Katheterlabores/Operationssaales abgegeben.

Anders als in diesem Beispiel kann auch vorgesehen sein, dass das flüssige Kältemittel über Löcher in den Kryoballon 3 eintritt, welche über die Länge des Kryoballons verteilt in der Wandung des Katheters 2 angeordnet sind.

Figur 3 zeigt einen Querschnitt der Figur 2 entlang der Schnittlinie A-A. Von innen nach außen zeigt Figur 3 den J-wire 7, das Lumen 6 zur Führung des J-wires, das Lumen 5 zur Führung des flüssigen Kältemittels, das Lumen 4 zur ersten Befüllung des Kryoballons 3 mit Druckluft und zur anschließenden Abführung des gasförmigen Kältemittels.
Die mit dem Drucksensor 14 und den Elektroden 16 verbundenen elektrischen Leitungen 15 verlaufen in dieser Ausführungsform im Lumen für die Abführung des dampfförmigen Kältemittels (N₂O).

Figur 4 stellt eine Ausführungsform des Katheters 2 bzw. insbesondere des Kryoballons 3 dar. Um das Risiko zu vermindern, dass es während der Behandlung aufgrund eines Blutstaus vor dem Kryoballon 3 zu einer Unterversorgung der Niere und damit zu einem Niereninfarkt kommt, ist diese Ausführungsform des Kryoballons 3 nicht symmetrisch sondern entspricht der Form eines Zylindersektors. In der hier dargestellten Ausführungsform wird von dem Kryoballon 3 ein Kanal 8 frei gelassen, durch welchen auch im Behandlungsfall Blut in Richtung Niere strömt und diese mit dem nötigen Sauerstoff versorgt.
Der Kryoballon 3 besitzt in dieser Ausführungsform die Gestalt eines Zylindersektors, wobei die Zirkumferenz des Kryoballons einen Radius von mehr als 180°, bevorzugt mehr als 230° aufspannt. Wenn der Zylindersektor einen Radius von mehr als 180° aufspannt, kann gewährleistet werden, dass die gesamte Zirkumferenz der Gefäßwand mit dem Kryoballon 3 in Kontakt kommt, wenn der Kryoballon 3 während der Behandlung wenigstens einmal um die Längsachse um einen Mindestwinkel gedreht wird, welcher größer, als der Winkel ist, welchen der ausgesparte Zylindersektor des Kanals 8 aufspannt.

Vorzugsweise besitzt der Katheter 2 an einem proximalen Ende Markierung(en) 13, mit welchen festgestellt werden kann, ob die während der Behandlung durchgeführte Rotation um die Längsachse größer ist, als der von dem Kanal 8 aufgespannte Winkel. Auf diese Weise kann sichergestellt werden, dass die gesamte Zirkumferenz der renalen Arterie von dem Kryoballon 3 während der Behandlung berührt wird und die renalen perivaskulären Nerven damit auf der gesamten Länge der Arterie und der gesamten Zirkumferenz abladiert werden.

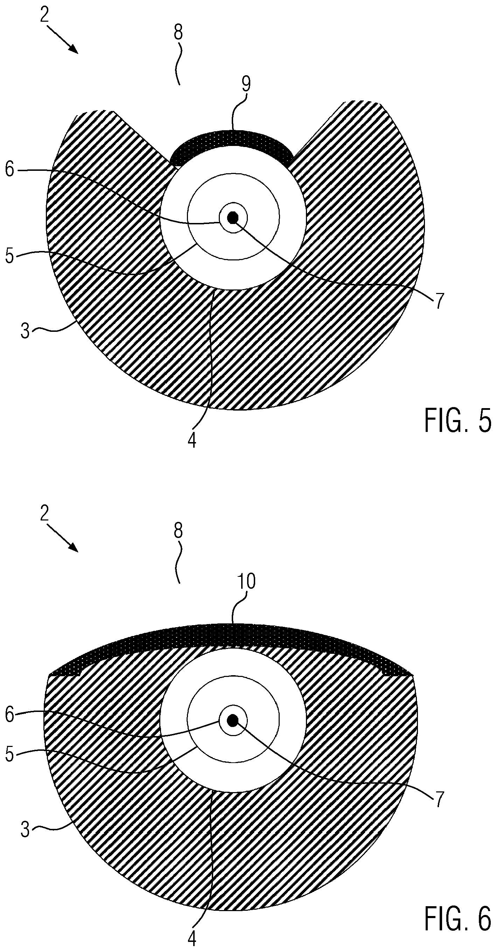

Figur 5 ist eine weitere Ausführungsform, bei der der Kryoballon 3 im Behandlungsfalle nicht die gesamte Innenwand der renalen Arterie berührt und einen Kanal 8 für das Blut freihält. Hierfür ist ein Streifen 9 eines wenig elastischen Materials auf den Katheter 2 vor und hinter dem Kryoballon 3 befestigt, z. B. geklebt. Dieser Streifen 9 verhindert ein Ausdehnen des Kryoballons 3 in dem Bereich des Streifens 9.
Gemäß einer bevorzugten Ausführungsform ist der Streifen 9 aus einem Material mit geringer Wärmeleitfähigkeit gefertigt oder es ist zusätzlich zu dem Streifen 9 ein isolierendes Material vorgesehen, welches verhindert, dass das durch den Kanal 8 strömende Blut im Behandlungsfalle gefriert oder verklumpt.

Figur 6 ist eine weitere Ausführungsform, die nicht Teil der Erfindung ist, bei der der Kryoballon 3 im Behandlungsfalle nicht die gesamte Innenwand der renalen Arterie berührt. In diesem Falle ist eine nicht oder wenig dehnbare Schicht 10 vorgesehen, welche auf der Oberfläche des Kryoballons 3 befestigt ist, oder die dehnbare oder gefaltete Oberfläche des Kryoballons 3 durch eine nicht oder wenig dehnbare und/oder nicht entfaltbare Schicht ersetzt. Auch auf diese Art und Weise kann ein Kanal 8 freigehalten werden und es wird während der Behandlung nie die komplette Blutzufuhr der Niere durch den Kryoballon 3 blockiert, sodass das Risiko eines Niereninfarktes deutlich reduziert ist. Wie in der in Figur 5 dargestellten Ausführungsform kann auch bei dieser Ausführung vorgesehen sein, dass die Schicht 10 aus einem Material mit geringer Wärmeleitfähigkeit gebildet ist.

## Patentansprüche

1. Medizinisches Gerät (1) zur Denervierung renaler perivaskulärer Nerven mit
einem Katheter (2), der einen flexiblen Schaft zum Einführen in die renale Arterie eines Patienten umfasst, wobei der Schaft ein inneres Lumen zum Zuführen eines Kältemittels und einen am distalen Ende des Schaftes angeordneten Kryoballon (3) aufweist, wobei
auf dem Kryoballon (3) wenigstens eine bipolare Elektrode (16) zur Abgabe eines elektrischen Impulses zur Stimulation der perivaskulären Nerven angeordnet ist,
**dadurch gekennzeichnet, dass** der Kryoballon (3) dazu ausgebildet ist, im Anwendungsfall die Wand der renalen Arterie auf einer Länge von mehr als 2,5 cm durchgängig zu berühren und zu kühlen und dadurch bei entsprechender Temperatur und Anwendungszeit auf einer Länge von mindestens 2,5 cm und über wenigstens 230° seiner Zirkumferenz durchgehend und gleichzeitig zu abladieren, wobei an der Außenseite des Kryoballons (3) ein Kanal angeordnet ist, der im Behandlungsfall für geringen Blutfluss ausgelegt ist und zwischen 10 und 40 % der Zirkumferenz entspricht.

2. Medizinisches Gerät (1) nach Anspruch 1, **dadurch**
**gekennzeichnet, dass** der Katheter (2) im Anwendungsfall mit einer Kältemittelquelle (12) und einer Steuerung (11) für die Kältemittelquelle (12) verbunden ist, wobei die Kältemittelquelle bzw. die Zuführung des Kältemittels in den Kryoballon (3) in Abhängigkeit von einem Blutdruckwert eines Patienten gesteuert wird.

3. Medizinisches Gerät (1) nach Anspruch 1 oder 2, **dadurch gekennzeichnet, dass** der Katheter (2) mit einem Drucksensor (14) zur Bestimmung des Blutdrucks verbunden ist.

4. Medizinisches Gerät (1) nach Anspruch 2, **dadurch gekennzeichnet, dass** die Steuereinheit (11) mit der wenigstens einen bipolaren Elektrode (16) zur Abgabe eines elektrischen Impulses zur Stimulation der perivaskulären Nerven verbunden ist, wobei die Steuereinheit (11) bei Feststellung einer Blutdruckerhöhung um einen vorbestimmten Grenzwert, die auf eine Anregung der perivaskulären Nerven durch die wenigstens eine Elektrode (16) folgt, einen Kältemittelfluss zu dem Kryoballon startet oder aufrecht erhält.

5. Medizinisches Gerät (1) nach Anspruch 2, **dadurch gekennzeichnet, dass** die Steuereinheit (11) mit der wenigstens einen Elektrode (16) zur Abgabe eines elektrischen Impulses zur Anregung der perivaskulären Nerven verbunden ist, wobei die Steuerung bei Feststellung dass eine Blutdruckerhöhung um einen vorbestimmten Grenzwert, als Reaktion auf eine Stimulation der perivaskulären Nerven durch die wenigstens eine Elektrode 16 nicht stattfindet, einen Kältemittelfluss zu dem Kryoballon (3) unterbricht.

6. Medizinisches Gerät (1) nach Anspruch 2, **dadurch gekennzeichnet, dass** die Steuereinheit (11) einen Testmodus aufweist, bei dem der Kryoballon (3) auf eine Temperatur gekühlt wird, bei der die Reizleitung der renalen perivaskulären Nerven gedämpft oder unterbunden wird, eine Ablation der renalen perivaskulären Nerven jedoch nicht stattfindet.

7. Medizinisches Gerät (1) nach Anspruch 2, **dadurch gekennzeichnet, dass** die Läsionstiefe über eine Impedanzmessung mit Hilfe der Elektroden und der Steuerung durchgeführt wird, wobei die Ablation der renalen perivaskulären Nerven beendet wird, wenn ein vorgegebener Wert für die Ermittlung der Läsionstiefe über die Impedanzmessung ermittelt wurde.

8. Medizinisches Gerät (1) nach Anspruch 2, **dadurch gekennzeichnet, dass** die Behandlung nach kumulierten Ablationszeit von 18 Minuten beendet wird.

9. Medizinisches Gerät (1) nach einem der vorangehenden Ansprüche, **dadurch gekennzeichnet, dass** wenigstens 2 Elektroden (16) auf der Außenseite des Kryoballons (3) angeordnet sind.

10. Medizinisches Gerät (1) nach den vorhergehenden Ansprüchen, wobei der Kanal (8) kälteisoliert ist.

## Claims

1. A medical device (1) for the denervation of renal perivascular nerves, having:
a catheter (2) which comprises a flexible shaft for introduction into a patient's renal artery, wherein the shaft has an inner lumen for supplying a refrigerant and a cryoballoon (3) disposed on the distal end of the shaft, wherein
at least one bipolar electrode (16) is disposed on the cryoballoon (3) in order to deliver an electrical impulse for the stimulation of the perivascular nerves,
**characterized in that** the cryoballoon (3) is configured such that when in use, it contacts and cools the wall of the renal artery over a continuous length of more than 2.5 cm and thereby, at an appropriate temperature and application time, is configured to continuously and simultaneously ablate over a length of at least 2.5 cm and over at least 230° of its circumference, wherein a channel is disposed on the outside of the cryoballoon (3) which is configured for a small blood flow during treatment and which corresponds to between 10 % and 40 % of the circumference.

2. The medical device (1) as claimed in claim 1,
**characterized in that** when in use, the catheter (2) is connected to a refrigerant source (12) and a controller (11) for the refrigerant source (12), wherein the refrigerant source or the supply of the refrigerant to the cryoballoon (3) is controlled as a function of the patient's blood pressure.

3. The medical device (1) as claimed in claim 1 or claim 2, **characterized in that** the catheter (2) is connected to a pressure sensor (14) for determining the patient's blood pressure.

4. The medical device (1) as claimed in claim 2, **characterized in that** the control unit (11) is connected to the at least one bipolar electrode (16) for the delivery of an electrical impulse for the stimulation of the perivascular nerves wherein, when an increase in blood pressure by a predetermined limiting value following a stimulation of the perivascular nerves by the at least one electrode (16) is detected, the control unit (11) commences or maintains a flow of refrigerant to the cryoballoon.

5. The medical device (1) as claimed in claim 2, **characterized in that** the control unit (11) is connected to the at least one electrode (16) for the delivery of an electrical impulse for the stimulation of the perivascular nerves wherein, when an increase in blood pressure by a predetermined limiting value as a reaction to a stimulation of the perivascular nerves by the at least one electrode (16) is not detected, the controller interrupts a flow of refrigerant to the cryoballoon (3).

6. The medical device (1) as claimed in claim 2, **characterized in that** the control unit (11) has a test mode, during which the cryoballoon (3) is cooled to a temperature at which the conduction of stimuli by the renal perivascular nerves is attenuated or suppressed, but an ablation of the renal perivascular nerves does not take place.

7. The medical device (1) as claimed in claim 2, **characterized in that** the lesion depth is measured by means of an impedance measurement with the aid of the electrodes and the controller, wherein the ablation of the renal perivascular nerves is terminated if a prescribed value for the determination of the lesion depth has been determined by means of the impedance measurement.

8. The medical device (1) as claimed in claim 2, **characterized in that** treatment is ended after a cumulative ablation period of 18 minutes.

9. The medical device (1) as claimed in one of the preceding claims, **characterized in that** at least two electrodes (16) are disposed on the outside of the cryoballoon (3).

10. The medical device (1) as claimed in the preceding claims, wherein the channel (8) is insulated against cold.

## Revendications

1. Appareil médical (1) pour dénerver des nerfs périvasculaires rénaux avec un cathéter (2) qui comprend une tige flexible pour introduire dans l'artère rénale d'un patient, la tige présentant une lumière intérieure pour amener un cryogène et un cryoballon (3) placé à l'extrémité distale de la tige, cependant qu'au moins une électrode bipolaire (16) est placée sur le cryoballon (3) pour délivrer une impulsion électrique pour la stimulation des nerfs périvasculaires, **caractérisé en ce que** le cryoballon (3) est configuré pour toucher en continu et pour refroidir la paroi de l'artère rénale sur une longueur de plus de 2,5 cm, en cas d'application, et pour ainsi effectuer une ablation simultanément et en continu, pour une température et un temps d'utilisation correspondant, sur une longueur d'au moins 2,5 cm et sur au moins 230° de sa circonférence, cependant qu'un canal qui est conçu, en cas de traitement, pour un flux sanguin faible et qui correspond à entre 10 et 40% de la circonférence, est placé sur le côté extérieur du cryoballon (3).

2. Appareil médical (1) selon la revendication 1, **caractérisé en ce que** le cathéter (2) est relié, en cas d'application, à une source de cryogène (12) et à une commande (11) pour la source de cryogène (12), cependant que la source de cryogène ou l'alimentation du cryogène dans le cryoballon (3) est commandée en fonction d'une valeur de pression artérielle d'un patient.

3. Appareil médical (1) selon la revendication 1 ou 2, **caractérisé en ce que** le cathéter (2) est relié à un détecteur de pression (14) pour déterminer la pression artérielle.

4. Appareil médical (1) selon la revendication 2, **caractérisé en ce que** l'unité de commande (11) est reliée à la au moins une électrode bipolaire (16) pour délivrer une impulsion électrique pour la stimulation des nerfs périvasculaires, cependant que l'unité de commande (11), lorsqu'une augmentation de la pression artérielle d'un seuil prédéterminé qui suit une excitation des nerfs périvasculaires par la au moins une électrode (16) est constatée, démarre ou maintient un flux de cryogène vers le cryoballon.

5. Appareil médical (1) selon la revendication 2, **caractérisé en ce que** l'unité de commande (11) est reliée à la au moins une électrode bipolaire (16) pour délivrer une impulsion électrique pour la stimulation des nerfs périvasculaires, la commande interrompant le flux de cryogène vers le cryoballon (3) s'il est constaté qu'une augmentation de la pression artérielle d'un seuil prédéterminé en tant que réaction à une stimulation des nerfs périvasculaires par la au moins une électrode (16) n'a pas lieu.

6. Appareil médical (1) selon la revendication 2, **caractérisé en ce que** l'unité de commande (11) présente un mode de test pour lequel le cryoballon (3) est refroidi à une température pour laquelle la conduction cardiaque des nerfs périvasculaires est atténuée ou interrompue, une ablation des nerfs périvasculaires rénaux n'ayant toutefois pas lieu.

7. Appareil médical (1) selon la revendication 2, **caractérisé en ce que** la profondeur de lésion est exécutée par une mesure de l'impédance à l'aide des électrodes et de la commande, cependant que l'ablation des nerfs périvasculaires rénaux est terminée si une valeur prédéfinie pour la détermination de la profondeur de la lésion a été détectée par la mesure de l'impédance.

8. Appareil médical (1) selon la revendication 2, **caractérisé en ce que** le traitement est terminé après un temps cumulé d'ablation de 18 minutes.

9. Appareil médical (1) selon l'une des revendications précédentes, **caractérisé en ce qu'**au moins 2 électrodes (16) sont placées sur le côté extérieur du cryoballon (3).

10. Appareil médical (1) selon l'une des revendications précédentes, **caractérisé en ce que** le canal (8) est isolé contre le froid.
